# EUROPEAN PATENT SPECIFICATION

(11) **EP 3 341 873 B1**
(45) Date of publication and mention of the grant of the patent: **18.06.2025**
(21) Application number: 16781541.4
(22) Date of filing: 11.08.2016
(51) Int. Cl.: G16H 40/67

(54) **SYSTEM AND METHOD FOR AIDING AN OPERATOR IN AN EMERGENCY SITUATION INVOLVING A PATIENT**
SYSTEM UND VERFAHREN ZUR UNTERSTÜTZUNG EINES BEDIENERS BEI EINER NOTSITUATION MIT EINEM PATIENTEN
SYSTÈME ET PROCÉDÉ POUR AIDER UN OPÉRATEUR DANS UNE SITUATION D'URGENCE IMPLIQUANT UN PATIENT.

(30) Priority: 28.08.2015 IT UB20153285
(43) Date of publication of application: 04.07.2018
(73) Proprietor: INMM S.r.l., 44122 Ferrara (IT)
(72) Inventor: MENGHINI, Sabrina, 44121 Ferrara (IT)
(74) Representative: Conti, Marco
(86) International application number: PCT/IB2016/054842
(87) International publication number: WO 2017/037558

(56) References cited:
- WO-A1-2014/116968
- WO-A1-2015/039249
- WO-A2-2015/071847
- US-A1- 2002 065 682
- US-A1- 2013 035 581

## Description

### Technical field

This invention relates to a system and a method for aiding an operator in an emergency situation involving a patient.

The system comprises different components which, if suitably integrated, are capable of facilitating cooperative rescue work and reducing the likelihood of errors.

### Background art

In particular, the system, which is applicable to any emergency situation (accidents, mass disasters as well as management of emergencies arising out of mass migration), provides support in the decision-making process of the operator with regard to the therapeutic procedures to apply based on the level of severity of the patient's conditions (derived by means of at least one diagnostic algorithm, also known as triage) and based on the professional profile of the operator. Another object of this invention is a method for aiding an operator in an emergency situation involving a patient. Also an object of this invention is a computer program comprising instructions which are executable by a computer and which, when executed by the computer, allow the computer to implement the method. A yet further object of this invention is a hardware device to embody the system.

The hardware includes an embedded wearable computational device provided with suitable sensors, which collects information regarding the patient's health and the surrounding environment and which can communicate this information, after processing it if necessary.

Known in the prior art, e.g. from WO2014/116968 and WO2015/039249, are emergency health care support systems and methods which perform a pre-diagnostic analysis (triage) of patients and assign a priority order to the patients based on the urgency of their need for care.

For example, US 2015066521 describes a centralized system for sharing and organizing the information relating to the health conditions of patients in an emergency department in order to coordinate health care operators and allow them to attend to patients in the greatest need of care.

This system has several disadvantages, however. First of all, the system is applicable only in a hospital context, because it is centralized. Also, according to the method, the level of severity must be assessed, and the necessary treatment allocated, by qualified health care operators (for example, physicians and nurses). Secondly, the system described does not provide the operator with support in deciding what first aid procedure should be adopted based on the patients conditions.

US 2013030825 describes a system and a method for automatically assessing the level of severity of a patient at a first aid point. The system involves applying one or more sensors to a patient in order to detect certain basic biometric parameters and to automatically assign treatment priorities, thereby reducing the time needed to attend to patients in more serious conditions and at the same time optimizing the efforts of the operators concerned.

However, this system, too, is applicable only in a hospital context and requires aid to be provided only by qualified operators. Also, the system described does not provide the operator with any decision-making support regarding the first aid procedure to be adopted based on the patient's conditions. Further, the biometric parameters detectable by the sensors applied to the patient are only partial and are insufficient to effectively outline the patient's clinical state. Moreover, the biometric parameters detectable do not take into account the competencies of the operator and the technologies available.

WO2015071847 describes a method and a system for automatically evaluating a patient's level of severity. The system involves applying sensors to a patient to detect his/her biometric parameters and automatically assigning a level of severity and priority to his/her need for care. The system provides decision support by suggesting the most appropriate course of action to be taken based on the patient's conditions. It does not, however, make any distinction between health-care operators based on respective competencies (lay volunteers or qualified operators). As a result, operators may find themselves in a situation where they are required to provide treatment for which they have not received adequate training. Further, the system involves detecting biometric parameters to assign the level of severity to patients without, however, taking into account the competence of operators and the technology available to them in the field. Thus, operators may find themselves in a situation where they are required to detect biometric data for which they have not received adequate training or for which suitable equipment is not available. Moreover, although the system is applicable also in a non-hospital context, it does not envisage the possibility of arranging and requesting immediate transport to suitably equipped health-care facilities.

### Disclosure of the invention

The aim of this invention is to provide an emergency support system and method to overcome the above mentioned disadvantages of the prior art.

More specifically, this invention has for an aim to provide an emergency support system and method, allowing automatic assessment of a patient's level of severity, automatically assigning a level of severity and treatment priority, reducing the time needed to attend to patients whose conditions are more serious and coordinating the efforts of the operators involved.

A further aim of the invention is to provide an emergency support system and method which meet the need for integrating different health-care technologies, simplifying and facilitating operations in different (emergency) care settings.

A further aim of the invention is to propose an emergency support system and method which can select one or more therapeutic treatment procedures based on the patient's conditions and on the competencies of the operator involved (if necessary, coordinated from a control station), making rescue operations easier and reducing the likelihood of human error.

Another aim of the invention is to provide an emergency support system and method which envisages the possibility of arranging and requesting immediate transport to health-care facilities equipped to treat the patient in serious conditions.

A yet further aim of the invention is to provide one or more computer program and one or more appliances which can achieve the above mentioned aims.

These aims are fully achieved by the emergency support system, method, computer program and appliances forming the object of this invention and as characterized in the appended claims.

The system for aiding an operator in an emergency situation involving a patient according to this invention comprises at least one portable electronic appliance, further referred to as TAG which can be associated with the patient.

The TAG comprises one or more sensors for detecting biometric parameters of the patient.

The TAG is capable of processing the information collected. The TAG is also configured to highlight a reference code (for example a colour) representing the severity of the patient's conditions. The TAG is also configured to communicate the information collected (in effect, it is a data exchange module).

The system also comprises at least one mobile device, for example a tablet or a smart phone, or other electronic device of the type known as "smart device". The mobile device can be connected wirelessly to the TAG to receive the biometric parameters, store them in a memory and/or communicate them. The mobile device has a display and a processor programmed (in turn) to process the biometric parameters received according to at least one diagnostic algorithm for deriving a patient's level of severity.

The devices can cooperate with each other independently, each with the aim of supporting a specific human operator, supported by the system, by a communications infrastructure and by a control and supervision station.

Preferably, the patient's level of severity is stored in the memory of the device or in a remotely accessible data bank (supported by an adequate communications infrastructure). For example, the data bank resides in a server which defines a control and supervision station.

Further, the patient's level of severity is assigned a unique identification code associated with the patient through the TAG.

The TAG has a display or other element visible from a distance and which can preferably be coloured with an interchangeable colour under the control of a processor of the TAG itself.

Moreover, if the biometric parameters change, the level of severity can be modified and reprocessed by the TAG, either independently or by interaction with the mobile devices.

Thus, the TAG is provided with a memory and a processor. The memory contains a program code representing a triage algorithm configured to derive a severity level of the patient as a function of a plurality of parameters, at least some of which are biometric parameters detected by the TAG itself.

The TAG is programmed to update the values of the biometric parameters detected after some time and consequently to update the level of severity by re-running the program code.

The system also comprises a data bank containing a plurality of codes for identifying the operators. The data bank also contains a plurality of professional profile categories for the operators, combined with the identification codes of each operator.

The mobile device receives the operator's identification code (when the operator logs in or is authenticated) and selects a therapeutic procedure from a plurality of predetermined therapeutic procedures stored. Selection occurs as a function of the professional profile category of the operator who has logged in from and is using the mobile device and, in combination, as a function of the severity level derived for the patient.

Thus, the system is particularly effective because it provides the operator with practical indications as to the course of action to be followed based on the operator's experience and available equipment, as well as on the severity of the patient's conditions.

The processor of the mobile device also access a memory (resident in the mobile device itself or in a local or remote server) containing a plurality of diagnostic algorithms defining different levels of accuracy. In this case, the processor of the mobile device is programmed to select one or more of the algorithms as a function of the professional profile category of the operator who is logged in.

This makes the system particularly precise and allows providing a pre-diagnosis calibrated on the experience of, and equipment available to, the operator, as well as on the biometric parameters (or bioparameters) detected.

The system also makes it possible to effectively use the results of pre-diagnoses already performed on a patient, defining a pre-diagnostic structure on different levels (tree-like structure).

Thus, the resources of the operator and of the system in general are optimized.

By saving and archiving the parameters detected and the results of the pre-diagnostic algorithms, the system reduces the likelihood of error and provides support also for hospital staff who have the possibility of accessing the stored data, for example by connecting up to the remote server (defining a control station) containing the data bank.

The control station, which can, if necessary, be placed on the site of the incident, is capable of receiving continuous feedback on the operations in progress in order to coordinate the operations (even automatically and/or with the aid of qualified personnel) and to act as data collection and check point.

The control station is not part of the claimed invention.

For this purpose, the system and the components making it up can use any communications protocol known in the state of the art in such a way as to guarantee a continuous and high level of connectivity between the operators in the field and the control station (whether local or remote). Communication is possible even in the absence of connectivity. Preferably, the control station is configured to interact with a satellite location system.

Preferably, the control station is programmed to generate a first type of map, displayed on a display of the control station and/or on the display units of the mobile devices (smart devices, for example).

The first type of map defines a geolocation of an incident site on a map of a territory. The first type of map provides the rescue worker with a general picture of the patient's location and allows identifying areas of responsibility and access routes to reach the patient.

Preferably, the control station is programmed to generate a second type of map, displayed on a display of the control station and/or on the display units of the mobile devices (smart devices, for example).

The second type of map defines a geolocation of an incident site on an orthophotograph.

The second type of map provides the rescue worker with a geographical picture of the incident site, including an analysis of the context of, and any post-incident changes in, the territory concerned.

Preferably, the control station is programmed to generate a third type of map, displayed on a display of the control station and/or on the display units of the mobile devices (smart devices, for example).

The third type of map defines a geolocation on a three-dimensional model of an incident site on an orthophotograph.

The third type of map allows the rescue worker to monitor the site on an automatic 3D layout for rescue work at different heights or in critical situations.

Thus, this description relates to a system, capable of contributing to improving life-saving rescue operations in the event of incidents or major disasters, which can be used by civilian and/or military operators and which is adaptable to a variety of emergency settings.

The system has the advantage of facilitating and coordinating rescue operations and extending operators' range of action. The system integrates specific health care protocols, relating to reference pathological findings, with a set of integrated hardware and software technologies which fulfil the function of providing decision-making support for the operator because they allow therapeutic steps amd progress to be followed, that is to say, which are capable of suggesting/indicating the most appropriate treatment.

The emergency action must take into account the training and capabilities of the rescue worker, using the basic rule that a situation must not be made worse by administering improper care. The action must differ according to the place and available equipment, distinguishing the first aid given on the site of the incident (pre-hospital environment) in the period immediately following the incident, from the casualty treatment proper, which is the responsibility of qualified personnel and which may be performed at a later stage (in a hospital environment).

At present, since the risk of emergency situations appears to be continuously on the increase, it is essential for more and more people to be able to provide incident victims with immediate aid. In this regard, large communities of lay and volunteer rescue workers have been set up over time, thanks to the widespread diffusion of an innovative culture of life-saving awareness which, however, has led to an expectation of commensurate preparation.

The interval of time which elapses from the occurrence of the incident to the arrival of proper rescue means and qualified personnel on the site of the incident is a factor of the utmost importance. Indeed, it is in this period time that the risk of complications becomes critical if not adequately dealt with. For this reason, the actions of the first people to reach a disaster scene are crucial and, in most cases, those very people are not specialists.

First aid means not only implementing life-saving measures, protecting a victim from further injury and danger, promptly reporting the case to organized emergency health care structures and arranging rapid transport to properly equipped facilities but also, and above all, following the therapeutic steps in attending to the injured victim.

In some cases, it may be impossible to attend to all the victims involved. In these cases, it may be necessary to determine an order of priority for the victims, establishing a priority for evacuation, rescue methods in terms of ambulance transport and patient's final destination. This is referred to as pre-diagnosis or triage, a medical emergency diagnostic action which must take into account the location and available means in every single situation. In some cases, victims in very serious conditions, not recoverable with the means available in the field, may need to be abandoned in order to save other victims who are more likely to survive. At times, the first people to reach the site have different professional profiles and competencies. In such cases, it may be necessary to determine a single course of action (uniform, standardized protocols) using technological supports which can step an operator through a procedure for rapidly recognizing the potential risks and identifying the diagnostic picture, the proper administration of suitable therapy and the patient's destination. That is because one of the fundamental standards is the accuracy of the action and of the clinical documentation to be made available, also with a view to managing and reducing the clinical risk, that is, exposure to clinical error due to incomplete communication of diagnostic and treatment information which precedes and must be integrated with hospitalization. The set of actions which constitutes the immediate care to be given to incident victims and which must be taken as quickly as possible is the responsibility not only of medical and emergency surgical (and hence qualified) operators but also of lay rescue workers who administer first aid, where this is within their capabilities, before other, more specific and decisive actions are taken. That is because the rescue team as a whole (which, besides rescue workers, includes doctors and other expert people located in different parts of the territory) must be placed in a position to cooperate properly in the context of the situation in the field, in order to be able to coordinate the action not only of a single rescue worker but also of all the rescue workers as a whole as efficiently and accurately as possible.

The approach to the patient in critical and emergency conditions has certain characteristics worthy of note.

On the one hand, it is a repetitive cycle of procedures creating the risk of lowering the level of attention of the operator involved and, on the other, require decisions, sometimes very important, to be made in highly stressful situations and based frequently on the acquisition (and use) of coded information and parameters.

To this must be added the fact that the available time is extremely limited, leading on many occasions to conflict between the need for data to be collected accurately and the need for decisions to be made very rapidly. The current context is characterized by:
- non-uniformity of the instruments used up to now (clinical reference cards on paper or simply scanned with software programs);
- low level of reliability of the instruments used (fragility of the paper, time needed for recording the information, illegibility, incompleteness of the information collected, interference with care procedures).

These are factors which the day-to-day experience of rescue workers has revealed to be of critical importance for approaching an injured victim in emergency conditions. These are basic actions which have a low conceptual or technical content but which can have an enormous influence on the outcome of the emergency process of rescuing a patient.

The information collected in the field is clinical data making up the knowledge base which will subsequently be used to build up the set of treatments and specialist services to be administered to the patient, that is to say, the hospitalization process.

One of the solutions to the critical factors mentioned above is to provide a picture representing the activities to be carried out (rescue workflows) for complex situations, where personnel taking part have predetermined roles and duties, and advising them as to what is the best practice for the activity in progress, what are the elements necessary for the steps of diagnosing and treating the patient and the circumstances which constitute limitations that interfere with these processes. Information becomes routine and constantly accessible and generates the therapeutic steps to be observed.

The result is a tree-like structure which integrates specific health care protocols, relating to reference pathological findings, with a set of integrated hardware and software technologies which fulfil the function of providing decision-making support for the operator in that they are capable of suggesting/indicating the most appropriate treatment.

In light of this, it should be noted that this description provides a computer program comprising instructions which are executable by the computer. These instructions, when executed by the computer, allow the computer to perform the following steps:
- receiving an identification code uniquely assigned to an operator;
- receiving one or more biometric parameters of a patient assisted by the operator;
- comparing the one or more biometric parameters received with respective predetermined ranges of values;
- deriving the patient's level of severity based on the one or more biometric parameters detected and at least one diagnostic algorithm of base level category,

The program is configured to access a memory containing a plurality of professional profile categories for the operator, where each identification code of each operator is associated with one of the professional profile categories.

The memory contains a plurality of predetermined therapeutic procedures. The processor of the mobile device is programmed to access the data bank and select the professional profile category corresponding to the identification code received, and to select at least one therapeutic procedure from the plurality as a function of the selected professional profile category and as a function of the patient's derived level of severity. The program can select one or more diagnostic algorithms from a plurality of diagnostic algorithms of a category higher than the base level category, the diagnostic algorithms being selectable by the operator based on the professional profile category of the operator himself/herself.

The claimed invention includes a computer program comprising a software for performing the steps, performed by the processor of the mobile device, when the computer program run on the mobile device of the system. Moreover, the claimed invention includes another computer program comprising a software for performing the steps, performed by the processor of said at least one electronic appliance, when run on said at least one electronic appliance of the system.

Thus, this description provides an appliance for aiding an operator in an emergency situation involving a patient.

The appliance comprises:
- at least one portable electronic appliance (or TAG) positionable at the patient and comprising one or more sensors used to detect one or more biometric parameters of the patient; the TAG is an appliance which is operatively associated with the patient and is configured to provide information about the patient and to communicate to the operator information regarding the patient's identity, state of health and progress of his/her level of severity, by displaying a reference code (for example a colour); the TAG is configured to be queried by the operators and to passively supply information; the TAG is also configured to operate actively by autonomously (automatically) sending the sensed data to the operators (for example to the remote server of the system or of a hospital);
- at least one mobile device connectable wirelessly to the at least one portable electronic appliance to receive the biometric parameters detected by the at least one portable electronic appliance, having access to a first information medium for storing the biometric parameters detected and having a display and a processor programmed to receive and process the biometric parameters received, where the first information medium contains at least one diagnostic algorithm to derive a level of severity of the patient's condition, and wherein the processor is configured to perform the diagnostic algorithm as a function of the biometric parameters received;
- a second information medium accessible by the at least one mobile device and containing a plurality of identification codes, each uniquely assigned to an operator.

The second information medium contains a plurality of professional profile categories for the operator, wherein each identification code of each operator is associated with one of the professional profile categories.

The mobile device comprises a data exchange interface configured to receive the operator's identification code, where the first information medium contains a plurality of predetermined therapeutic procedures, and where the processor of the mobile device is programmed to access the second information medium and select the professional profile category corresponding to the identification code received, and to select at least one therapeutic procedure from the plurality as a function of the selected professional profile category and as a function of the derived level of severity of the patient's condition.

The system according to this description is preferably configured to allow an operator to retrieve, locally or remotely or by querying the TAG, the information relating to the level of severity (and to the biometric parameter detected). The system is also configured to allow the same operator, or other operators, to process and modify the information associated with the injured persons, including the level of severity, to vary it based on the progress of the rescue operations.

Preferably, the TAG (or each TAG) is configured to periodically communicate its geo-referenced position in the field of action and its state of activity to the operations control station and can receive information and instructions therefrom.

The system is configured to manage patient evacuation in the field based on one or more of the following factors:
- level of severity of the patients;
- rescue means and equipment available on site or called in (also arranged beforehand as a function of the patients' level of severity);
- following an explicit request, communicating the outcomes of operations by the operators.

The remote server, or control station, is programmed to generate notice events which can be addressed both to operators working at the control station and to operators busy with rescue operations on site.

More specifically, the step of sending notices regards the following, alternatively or in combination:
1. the need for additional resources (both human and material) in the field;
2. the reporting of alert values (outside normal permitted values) regarding patients being assisted and still in the field;
3. the addition of another patient to be assisted.

This description also provides a computer program comprising a software program for performing the steps of the method according to one or more of the aspects of this description (when run on the mobile device of the system according to one or more of the aspects of this description).

Using this program, the operator can do his/her own work and, at the same time, can be supported by the system to work in cooperation with other operators.

It should be noted that a further advantage of the system and method of this description is that of providing operators with an overall view of the state of the operations in progress and of the operators involved. According to another aspect, this description provides an interface element obtained by means of a software program and, if necessary, also hardware elements.

In functional terms, this interface is placed between the mobile device (or the tag) and the server (which acts as a hub) to manage communications between the mobile device and the server.

The interface element is configured to perform the following actions:
- tracing (identifying) and verifying the available transmission channels;
- selecting the fastest transmission channel;
- adapting to the selected transmission channel the information packets to be transmitted.

These actions are carried out in real time.

In a possible embodiment, the hardware of the interface element might include communication interfaces of the different channels, which might not already be present in the mobile device (mobile phone or tablet). Without the additional hardware, the functions are limited to tracing and selecting the communication channels which are compatible with the hardware available in the mobile device.

Whatever the case, the interface element is programmed to process the data to be transmitted in order to adapt the amount of data to the band and type of channel available, or selected.

In one embodiment, the system might include the interface element. It is understood, however, that this description is also extended to the interface element as such.

### Brief description of the drawings

These and other features of the invention will become more apparent from the following detailed description of a preferred, non-limiting embodiment, with reference to the single accompanying drawing which schematically represents the system according to this description.

### Detailed description of preferred embodiments of the invention

The numerals in the drawing denote the following:
- the numeral 1, the system;
- the numeral 2, an operator or rescue worker;
- the numeral 3, the patient assisted;
- the numeral 4, a mobile device such as, for example, a tablet or a smart phone;
- the numeral 5, a portable electronic appliance applicable to the patient 3 (this portable electronic appliance being also referred to as TAG in this description);
- the numeral 6, one or more sensors the TAG 5 is provided with;
- the numeral 7, a communications interface the TAG 5 is provided with for wireless connection to the mobile device 4 (or to a remote server);
- the numeral 8, a display of the mobile device 4;
- the numeral 9, a server (remote), or control station.

An analysis of the main emergency topics crossed with guide lines made it possible to establish which parameters are the first indicators of the pathological conditions of interest and to establish whether these are fixed or change over time and according to the clinical picture. The parameters were selected as a function of the possibility of being detectable by all the professional profiles of the rescue workers involved in the emergency rescue operations (lay volunteer workers or professionals, that is, nurses or physicians) even if for some of them, especially in the later stages of rescue, it is compulsory to refer to professional levels of specific competence.

The parameters were divided into common parameters and parameters belonging to the single classes of professionals. The pathological conditions of interest are listed below:
- multiple trauma, with special emphasis on bleeding;
- acute coronary syndromes;
- stroke;
- non-cardiogenic respiratory insufficiency
- epidemiological surveillance;
- chronic kidney insufficiency up to dialysis.

Below is a summary of the results achieved by the system according to this description.

The parameters are summarized in Table 1 and can be divided into four classes of sensors, that is to say, the level of technology or competence required to identify the condition or symptom indicated.

Level 1 corresponds to vital parameters and non-instrumental clinical signs which do not require the use of technological instruments and advanced competencies to be identified and which are therefore accessible to a wide range of professionals and operators. Level 2 necessitates pocket instruments and identifies vital parameters which do not require competencies more specific than those required for level 1, with the exception of measurement of blood glucose and ETCO2 (for which advanced competencies are needed). Level 3 necessitates using complex technological equipment, which is not always available, such as POCT (point of care testing) equipment for measuring blood values. Lastly, level 4 involves little use of technological equipment and a high level of clinical competence, referred to as clinical acumen. This level requires advanced competence and can thus be implemented by health-care professionals with such competencies.

**Table 1: Biometric parameters detectable as a function of sensor class (and hence of the operator's professional profile category)**

| **signs/symptoms** | **Sensor class** | **SCA** | **Respiratory insuff.** | **Stroke** | **Trauma** | **Shock** | **Sepsis** | **OTHER** |
|---|---|---|---|---|---|---|---|---|
| FC | 1 | >100 | >100 | | >100 | >100 | >100 | |
| FR | 1 | >20 | >30 | | >30 | >30 | <30 | |
| GCS | | | <12 | | <8 | <12 | | |
| AVPU | 1 | | | | | | | |
| Capillary refill | 1 | | | | >2" | >2" | >2" | |
| Cyanosis | 1 | | Yes | | | Yes | | |
| Paleness | 1 | Yes | | | | Yes | Yes | |
| Diaphoresis/blotching | 1 | | Yes | | | | Yes | |
| PA | 2 | <90 | | | <80 | <80 | <90 | |
| SaO2 | 2 | <90% | <87% | | <95% | | | |
| ETCO₂ | 2 | | <45 | | | | | <10 |
| Blood glucose | 2 | | | | | | >140 | <70 |
| T° | 2 | | | | | | >38.5°C | |
| Troponin | 3 | >10 | | | | | | |
| PaO₂ | 3 | | <60 | | | | | |
| PaCO₂ | 3 | | >45 | | | | | |
| Respiratory distress | 4 | | | | | | | |
| Sensory disorders | 4 | | | | | | | |
| Oxygen therapy response | 4 | | | | | | | |
| Shock class 1 | 4 | | | | | | | |
| Shock class 2 | 4 | | | | | | | |
| Shock class 3 | 4 | | | | | | | |
| Shock class 4 | 4 | | | | | | | |
| Typical thoracic pain | 4 | Yes | | | | | | |
| Cold sweating | 4 | Yes | | | | | | |
| External haemorrhage | 4 | | | | Yes | | | |

Based on the data shown above it possible to create a computer program which allows facilitating the registration (recording) in different contexts.

Using a portable mobile device (such as a tablet), the operator can type in his/her unique identification code and then measure the parameters which are detectable based on sensor class, on his/her competencies and on available technology.

The system helps the operator recognize potential risks and rapidly identify the diagnostic picture, thereby allowing the most suitable treatment to be administered promptly and also allowing the patient's next destination to be chosen correctly. The system allows minimizing the clinical risk even in very difficult situations where an operator's threshold of attention is likely to be low.

The computer program, supported by the mobile device, accesses a data bank which collects all the above mentioned pathological findings and suggests appropriate therapeutic procedures, acting as a decision-making support for the operator, while observing the therapeutic steps and the constantly updated progress thereof. In particular, the computer program is capable of recognizing the operator's professional profile category and thus to permit or exclude the therapeutic procedures according to the operator's competencies.

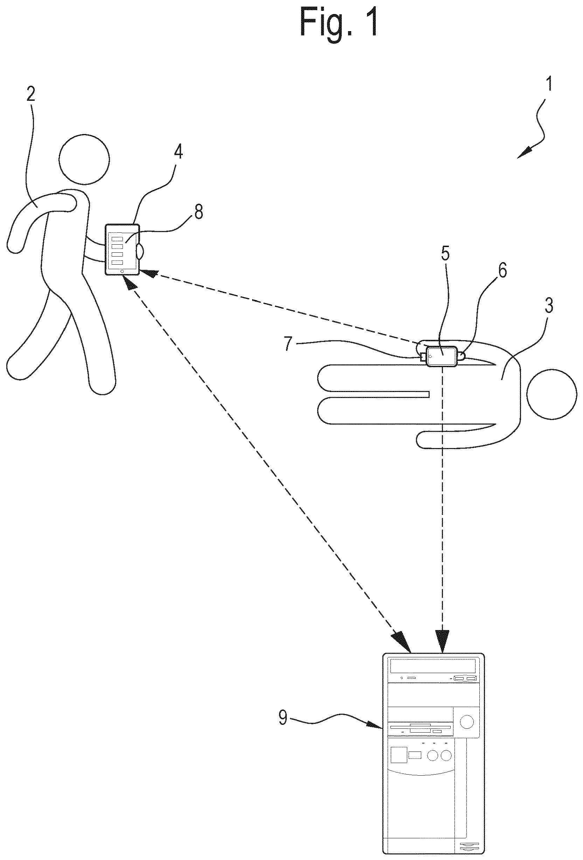

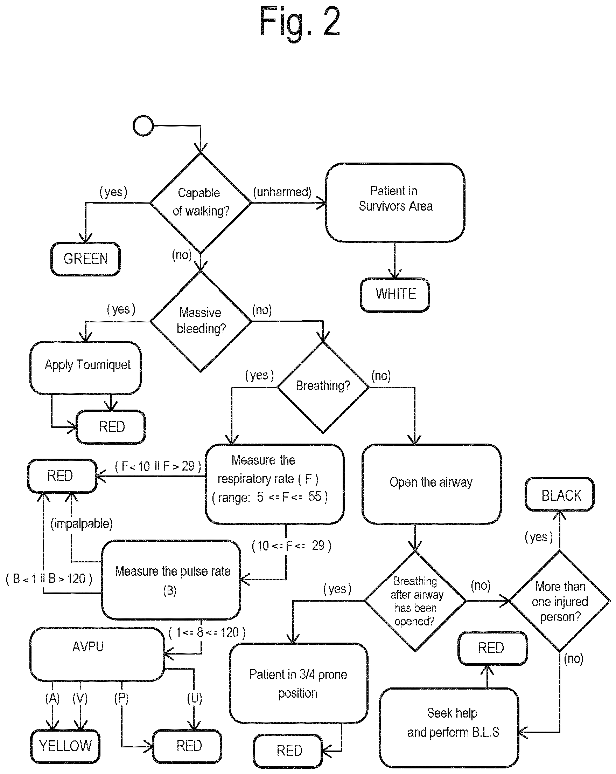

The therapeutic procedures are provided by the program based on the operator's professional category and the patient's severity level. The patient's severity level is derived by means of at least one diagnostic (triage) algorithm corresponding to the operator's professional profile. Figure 2 shows a base level diagnostic (triage) algorithm shared by both the less expert professional profile and the more expert professional profiles. Using biometric parameters measurable by the operator and non-instrumental clinical signs detectable by an operator who does not possess advanced competencies, the algorithm determines the patient's severity level. In particular, the patient's biometric parameters and clinical signs assessed by this algorithm are the following: walking capability, presence of massive bleeding, presence of breathing and measurement of respiratory rate, measurement of pulse rate, AVPU.

Comparing the biometric parameters and clinical signs with corresponding predetermined values and standard responses allows calculating the patient's level of severity and associating the severity level with a corresponding colour, which then appears on the display of the mobile device. More in detail, red indicates the highest severity level, yellow a medium severity level and green the lowest severity level; black indicates that the patient is deceased and white that a victim does not need medical assistance. The severity levels are directly associated with the care priority levels, which allows optimizing rescue operations (and the resources deployed) in the event of incidents involving many people.

A second base level diagnostic (triage) algorithm is shared by both the less expert professional profile and the more expert professional profiles. Using biometric parameters measurable by the operator and non-instrumental clinical signs detectable by an operator who does not possess advanced competencies, the algorithm determines the patient's severity level. In particular, the patient's biometric parameters and clinical signs assessed by this algorithm are the following: presence of breathing and measurement of respiratory rate, assessment of radial pulse, assessment of capillary refill, assessment of mental state.

Comparing the biometric parameters and clinical signs with corresponding predetermined values and standard responses allows calculating the patient's level of severity and associating the severity level with a corresponding colour, which then appears on the display of the mobile device.

The system memory also has a diagnostic (triage) algorithm of a level higher than the base level and selectable by a more advanced professional profile (for example, a physician). Using biometric parameters measurable by the operator and non-instrumental clinical signs detectable by an operator (who does not possess advanced competencies), the algorithm determines the patient's health conditions and suggests more appropriate therapeutic procedures based on the operator's competencies and the patient's conditions. In particular, the patient's biometric parameters and clinical signs assessed by this algorithm are the following: presence of neurological deficit, penetrating trauma, proximal amputation, burns, measurement of respiratory rate, oxygen saturation, heart rate, blood pressure, GCS scale.

Comparing the biometric parameters and clinical signs with corresponding predetermined values and standard responses allows determining the patient's health conditions and associating the most appropriate therapeutic procedures, which can be displayed on the display of the mobile device. In particular, the therapeutic procedures provided for the different severity states of the patient include: intubation, tracheotomy, cricothyrotomy, administration of benzodiazepines, propofol and ketamine, pleural drainage and pericardiocentesis.

Below is a description of the hardware components of the system according to this description.

The apparatus forming the object of this invention comprises a mobile device which allows rescue workers to use one or more diagnostic algorithms and to locally coordinate all the other devices and to interface with the components which integrate the system.

The apparatus according to this invention comprises a portable electronic appliance positionable at the patient and which allows detecting biometric parameters through specific sensors, dynamically displaying the state of severity of the patient's conditions, tracking the patient's position and identifying him/her during the entire rescue process. It also assists the rescue worker in performing standard operational algorithms.

The apparatus according to the invention comprises a server. The server records and allows displaying the biometric parameters of the different patients, allows participating in smart, automatic coordination operations and instantaneously sharing knowledge of the scene, which becomes an important condition for managing and planning the operations regarding the incident, assigning tasks and organizing human and instrumental resources.

The server acts both as a communication and distributed coordination bus for the members of the rescue team (in particular, rescue workers both in and out of the field) using the Internet (with suitable security measures) and related enabling transmission channels, and as a software system which allows rescue workers taking part in operations from one or more operating stations (in various parts of the territory) to monitor, control and coordinate operations.

Thus, this description provides a system for aiding an operator in an emergency situation involving a patient.

The system comprises at least one portable electronic appliance (or TAG) positionable at the patient (preferably, permanently associated with the patient) and comprising one or more sensors used to detect one or more of the patient's biometric parameters.

Preferably, the portable electronic appliance has an element by which it can be fixed to the patient (for example, a clip applicable to a garment, or a bracelet and a collar).

The system also comprises at least one mobile device (for example, a tablet or smart phone) connectable wirelessly to the at least one portable electronic appliance to receive the biometric parameters detected by the at least one portable electronic appliance.

The mobile device has access to a memory for storing the biometric parameters detected and having a display for viewing them.

The mobile device has a processor programmed to receive and process the biometric parameters received, where the memory contains at least one diagnostic algorithm to derive a level of severity of the patient's condition, and where the processor is configured to perform the diagnostic algorithm as a function of the biometric parameters received.

The system comprises a data bank accessible by the at least one mobile device. For example, the data bank resides in a remote server.

The data bank contains a plurality of identification codes, each uniquely assigned to an operator. The data bank also contains a plurality of professional profile categories for the operator, where each identification code of each operator is associated with one of the professional profile categories.

The mobile device also comprises a data exchange interface configured to receive the operator's identification code. The memory contains a plurality of predetermined therapeutic procedures.

The processor of the mobile device is programmed to access the data bank and select the professional profile category corresponding to the identification code received.

The processor of the mobile device is also programmed to select at least one therapeutic procedure from the plurality as a function of the selected professional profile category and as a function of the patient's derived level of severity.

In the system, the mobile device may also be programmed to transmit to the data bank the biometric parameters detected by the portable electronic appliance.

The system may also comprise a server containing the data bank and in communication with the mobile device through a local or remote connection.

In the system, the memory of the at least one mobile device may contain a plurality of diagnostic algorithms of a category higher than the base category, the diagnostic algorithms being selectable by the operator based on the professional profile category of the operator himself/herself.

The biometric parameters detectable by an operator belonging to a professional profile category at base level are chosen from the following list: heart rate, respiratory rate, GCS scale, AVPU scale, capillary refill, cyanosis, paleness, diaphoresis/blotching.

The biometric parameters detectable by an operator belonging to a professional profile category at a level higher than the base level are chosen from the following list: blood pressure, oxygen saturation (SaO2), CO2 value at end of exhalation (ETCO2), blood glucose, body temperature, troponin, blood oxygen partial pressure, blood CO2 partial pressure, respiratory distress, sensory disorders, oxygen therapy response, shock classes from 1 to 4, typical thoracic pain, cold sweating, external haemorrhage.

In the system, the processor may be programmed to arrange and request immediate transport of patients to health facilities.

This description also provides a computer program comprising a software program for performing the steps of the method according to this description (comprising one or more of the features or steps described). In particular, the computer program is configured to perform the steps of the method when run on the mobile device of the system according to this description (comprising one or more of the features or steps described).

This description also provides a data storage device (for example a CD-ROM or other mass storage medium) readable by a computer and containing the above mentioned computer program.

This description also provides a data flow downloadable onto a computer and representing the above mentioned computer program.

With regard to the biometric parameters detectable by the sensors of the system and usable by the system in the diagnostic algorithm, attention is drawn to the following.

In one embodiment of it, the system detects (preferably in real time) at least the person's heart rate and blood oxygen saturation, that is to say, the system detects the person's monitoring data, including signals representing the person's heart rate and blood oxygen saturation.

In this embodiment, the diagnostic algorithms include criteria based on a comparison of the values detected for these parameters with corresponding predetermined reference values. These diagnostic algorithms are based on a combination of the parameters including at least the heart rate (Fc) and the (peripheral) blood oxygen saturation (SpO2).

In one embodiment, the diagnostic algorithms use, in combination with the heart rate (Fc) and the (peripheral) blood oxygen saturation (SpO2), at least one further parameter: and that is, the intensity of pulsation. The signal representing the "intensity of pulsation" parameter is derived by analysing an amplitude of a signal measured by a pulsimeter. The pulsimeter is a sensor designed to measure heart rate (Fc) and (peripheral) blood oxygen saturation (SpO2).

It should be noted that the portable electronic appliance 5 (TAG) is programmed to update periodically and automatically the result of performing the diagnostic algorithm. In one embodiment, the portable electronic appliance 5 (TAG) is, consequently, also programmed to automatically update the light signal displayed by the portable electronic appliance 5 itself as a function of the result of performing the diagnostic algorithm.

At each update, the processor of the portable electronic appliance 5 uses, in the diagnostic algorithm, the updated values of the parameters measured by the sensors fixed to the person the portable electronic appliance 5 is associated with.

It should also be noted that the fact that the system adapts the diagnostic algorithm to the professional profile allows operators who are normally non-medical to provide rapid and effective assistance in emergency contexts.

This allows more lives to be saved. The use of complex algorithms by non-experts would result in time being wasted. In this regard, it should be noted that, at least at a first stage, the algorithms imply setting one or more parameters by displaying questions which the rescue worker must answer using the mobile device 4.

In one embodiment, the parameters entered by the rescue worker are not updated or modified each time the algorithm is updated automatically by the portable electronic appliance 5 but, in one embodiment, the rescue worker may (after setting the parameters the first time by answering the questions) take further action and thus also update the parameters regarding his/her answers to corresponding questions formulated by the system (based on the algorithm itself).

In one embodiment, the portable electronic appliance 5 (TAG) is in communication with the remote server (directly or through the dedicated interface element but without necessitating the mobile device 4) to communicate in real time the state of health and the position of the person the TAG is associated with.

In one example embodiment, communication between the portable electronic appliance 5 and the remote server comprises two-way data exchange. The portable electronic appliance 5 receives from the server information relating to environmental factors, entered by users or derived automatically by the server by processing information previously received by the server from the portable electronic appliance 5. This makes it possible to (re)run the diagnostic algorithm taking these environmental factors (such as altitude or temperature) also into account.

It should be noted that the portable electronic appliance 5 is uniquely assigned to a person (patient) and acts as a "black box" recording that person's state of health.

In one embodiment, the system (and in particular, the portable electronic appliance 5) is configurable, in the sense that it automatically learns from the person wearing it, the information it needs to "self-calibrate" on the person's physiological parameters (that is to say, it detects the user's activities and the related vital parameters - at rest, at work, under stress -, calculates a specific weighted mean thereof for each user and, consequently, calculates reference thresholds/presence of disorders - exceeding which/in the presence of which - sound and visual alarms are triggered).

In other words, the system (and in particular, the portable electronic appliance 5) is programmed to vary automatically and adaptively the reference values of the parameters used in the diagnostic algorithms, as a function of the signals measured by the sensor or sensors associated with the person (that is to say, as a function of the biometric parameters over time or total parameters of the patient).

According to one aspect of this description, the system is designed to prevent health problems for the user wearing it (that is to say, to facilitate the job of the rescue worker who is made aware of the situation arising and, in the presence of risk, takes appropriate rescue actions).

In one embodiment, the server is programmed to detect (through the signals from the different potrable electronic devices 5 associated with corresponding persons) signals coming (simultaneously) from a plurality of persons (patients). The server can also be programmed to display in a single representation data representing that plurality of persons (for example, displaying the respective position and state of health, in terms of a summary result of the diagnostic algorithm, for example with the colour code).

In one embodiment, the system allows activating tasks, that is, profiles having corresponding reference thresholds (for example, construction site workers and relevant safety regulations, environmental restrictions) so that the system can be used as a suitable preventive tool for reducing the risks of accidents at the place of work. The server might also be programmed to statistically process the data collected as a function of such tasks. This might be useful, for example, to make improvements to the living and working environment, or to the corresponding labour regulations.

## Claims

1. A system (1) for aiding an operator (2) in an emergency situation involving a patient (3), comprising:
- at least one portable electronic appliance (5) positionable at the patient and comprising one or more sensors (6) configured to detect one or more biometric parameters of the patient (3);
- at least one mobile device (4) connectable wirelessly to the at least one portable electronic appliance (5) and configured to receive the biometric parameters detected by the at least one portable electronic appliance, the at least one mobile device (4) comprising access to a memory for storing the biometric parameters detected and comprising a display (8) and a processor programmed to receive and process the biometric parameters received, wherein the memory further contains at least one diagnostic algorithm to derive a level of severity of the patient's condition, and wherein the processor is configured to execute the at least one diagnostic algorithm as a function of the biometric parameters received, and to associate the severity level with a corresponding colour appearing on the display (8) of the mobile device (4);
- a data bank accessible by the at least one mobile device (4) and containing a plurality of identification codes, each uniquely assigned to an operator (2),
wherein:
- the at least one portable electronic appliance (5) comprises a memory and a processor, wherein the memory contains a program code representing a triage algorithm, run by the processor of said at least one portable electronic appliance and configured to derive the level of severity of the patient as a function of a plurality of parameters, at least some of which are the biometric parameters detected by the at least one portable electronic appliance (5);
- the data bank contains a plurality of professional profile categories for the operator, wherein each identification code of each operator (2) is associated with one of the professional profile categories;
- the at least one mobile device (4) comprises a data exchange interface configured to receive the operator's identification code, wherein the memory contains a plurality of predetermined therapeutic procedures, and wherein the processor of the mobile device (4) is programmed to access the data bank and select the professional profile category corresponding to the identification code received, and to select at least one therapeutic procedure from the plurality as a function of the selected professional profile category and as a function of the level of severity of the patient's condition derived by the mobile device, so as to provide the operator with practical indications as to the course of action to be followed,
wherein the at least one portable electronic appliance further comprises a display or an element visible from a distance which can be coloured with an interchangeable colour under the control of a processor of the portable electronic appliance to highlight a reference code representing the severity of the patient's conditions as derived by the portable electronic appliance.

2. The system according to claim 1, comprising a server containing the data bank and in communication with the mobile device through a local or remote connection.

3. The system according to any one of the preceding claims, wherein the biometric parameters detectable by an operator belonging to a professional profile category at base level are chosen from the following list: heart rate, respiratory rate, GCS scale, AVPU scale, capillary refill, cyanosis, paleness, diaphoresis/blotching.

4. The system according to any one of the preceding claims, wherein the biometric parameters detectable by an operator belonging to a professional profile category at a level above the base level are chosen from the following list: blood pressure, oxygen saturation (SaO2), CO2 value at end of exhalation (ETCO2), blood glucose, body temperature, troponin, blood oxygen partial pressure, blood CO2 partial pressure, respiratory distress, sensory disorders, oxygen therapy response, shock classes from 1 to 4, typical thoracic pain, cold sweating, external haemorrhage.

5. The system according to any one of the preceding claims, wherein the processor of the mobile device is programmed to arrange and request immediate transport of a patient to health facilities as a function at least of the derived level of severity of the patient's condition.

6. The system according to any one of the preceding claims, wherein the at least one portable electronic appliance (5) is programmed to periodically update the values of the biometric parameters detected and consequently to update the level of severity by re-running the program code.

7. A method for aiding an operator in an emergency situation involving a patient, comprising the following steps:
- receiving an identification code uniquely assigned to an operator;
- detecting one or more biometric parameters of a patient assisted by the operator, though a portable electronic appliance (5);
- receiving the one or more biometric parameters in a mobile device (4), wirelessly connected to the at least one portable electronic appliance (5) and including a memory containing at least one diagnostic algorithm to derive a level of severity of the patient's condition;
- comparing the one or more biometric parameters detected with respective predetermined ranges of values;
- in a processor of the mobile device (4), executing the at least one diagnostic algorithm as a function of the biometric parameters received,
and to associate the severity level with a corresponding colour appearing on the display (8) of the mobile device (4); **characterized in that**
- the portable electronic appliance (5) is provided with a memory and a processor and derives, though a triage algorithm run by the processor, the level of severity of the patient as a function of a plurality of parameters, at least some of which are the biometric parameters detected by the at least one portable electronic appliance (5); and
**in that** the method comprises the following step, performed by a processor of the mobile device (4):
- accessing a data bank containing a plurality of identification codes, each uniquely assigned to an operator (2), and a plurality of professional profile categories for the operator;
- selecting the professional profile category corresponding to the identification code received,
- selecting at least one therapeutic procedure from among a plurality of predetermined therapeutic procedures, as a function of the selected professional profile category and as a function of the level of severity of the patient's condition derived by the mobile device, so as to provide the operator with practical indications as to the course of action to be followed, wherein the at least one portable electronic appliance further comprises a display or an element visible from a distance which can be coloured with an interchangeable colour under the control of a processor of the portable electronic appliance to highlight a reference code representing the severity of the patient's conditions as derived by the portable electronic appliance.

8. The method according to claim 7, comprising a step of arranging immediate transport of patients to health facilities as a function at least of the derived level of severity of each patient's condition.

9. The method according to any one of claims 7 or 8, wherein the at least one portable electronic appliance (5) periodically updates the values of the biometric parameters detected and consequently updates the level of severity by re-running the program code.

10. A computer program comprising a computer-executable instructions for performing the steps by the processor of the mobile device, according to any one of claims 7 to 9, when run on the mobile device of the system according to any one of claims 1 to 6 and comprising a computer-executable instructions for performing the steps by the processor of said at least one electronic appliance, according to any one of claims 7 to 9, when run on said at least one electronic appliance of the system according to any one of claims 1 to 6.

11. A data storage device readable by a computer and containing the computer program according to claim 10.

12. A data flow downloadable onto a computer and representing the computer program according to claim 10.

## Patentansprüche

1. System (1) zur Unterstützung eines Bedieners (2) bei einer Notfallsituation mit einem Patienten (3), umfassend:
- mindestens ein tragbares elektronisches Gerät (5), das am Patienten positionierbar ist und einen oder mehrere Sensoren (6) umfasst, die dazu ausgelegt sind, einen oder mehrere biometrische Parameter des Patienten (3) zu erfassen;
- mindestens eine mobile Vorrichtung (4), die drahtlos mit dem mindestens einen tragbaren elektronischen Gerät (5) verbindbar und ausgelegt ist, um die von dem mindestens einen tragbaren elektronischen Gerät erfassten biometrischen Parameter zu empfangen, wobei die mindestens eine mobile Vorrichtung (4) Zugriff auf einen Speicher zum Speichern der erfassten biometrischen Parameter umfasst und eine Anzeige (8) und einen Prozessor umfasst, der programmiert ist, um die empfangenen biometrischen Parameter zu empfangen und zu verarbeiten, wobei der Speicher ferner mindestens einen Diagnosealgorithmus enthält, um einen Schweregrad des Zustands des Patienten abzuleiten, und wobei der Prozessor ausgelegt ist, um den mindestens einen Diagnosealgorithmus als Funktion der empfangenen biometrischen Parameter auszuführen und den Schweregrad mit einer entsprechenden Farbe zu assoziieren, die auf der Anzeige (8) der mobilen Vorrichtung (4) erscheint;
- eine Datenbank, auf die das mindestens eine mobile Vorrichtung (4) zugreifen kann und eine Vielzahl von Identifikationscodes enthält, die jeweils eindeutig einem Bediener (2) zugewiesen sind,
wobei:
- das mindestens eine tragbare elektronische Gerät (5) einen Speicher und einen Prozessor umfasst, wobei der Speicher einen Programmcode enthält, der einen Triage-Algorithmus darstellt, der von dem Prozessor des mindestens einen tragbaren elektronischen Geräts ausgeführt wird und dazu ausgelegt ist, den Schweregrad des Patienten als Funktion einer Vielzahl von Parametern abzuleiten, von denen mindestens einige die biometrischen Parameter sind, die von dem mindestens einen tragbaren elektronischen Gerät (5) erfasst werden;
- die Datenbank eine Vielzahl vom Berufsprofilkategorien für den Bediener enthält, wobei ein jeder Identifikationscode eines jeden Bedieners (2) mit einer der Berufsprofilkategorien assoziiert ist;
- die mindestens eine mobile Vorrichtung (4) eine Datenaustauschschnittstelle umfasst, die zum Empfangen des Identifikationscodes des Bedieners ausgelegt ist, wobei der Speicher eine Vielzahl von vorbestimmten therapeutischen Vorgehen enthält und wobei der Prozessor der mobilen Vorrichtung (4) programmiert ist, auf die Datenbank zuzugreifen und die Berufsprofilkategorie auszuwählen, die dem empfangenen Identifikationscode entspricht, und mindestens ein therapeutisches Vorgehen aus der Vielzahl als Funktion der ausgewählten Berufsprofilkategorie und als Funktion des Schweregrads des von der mobilen Vorrichtung abgeleiteten Zustands des Patienten auszuwählen, um dem Bediener praktische Hinweise über die zu befolgende Vorgehensweise zu geben, wobei das mindestens eine tragbare elektronische Gerät ferner eine Anzeige oder ein aus einer Entfernung sichtbares Element umfasst, das unter der Steuerung eines Prozessors des tragbaren elektronischen Geräts mit einer austauschbaren Farbe gefärbt werden kann, um einen Referenzcode hervorzuheben, der die Schwere der Zustände des Patienten darstellt, wie sie durch das tragbare elektronische Gerät abgeleitet werden.

2. System nach Anspruch 1, umfassend einen Server, der die Datenbank enthält und über eine lokale oder entfernte Verbindung mit der mobilen Vorrichtung in Kommunikation steht.

3. System nach einem der vorhergehenden Ansprüche, wobei die biometrischen Parameter, die von einem Bediener erfassbar sind, der zu einer Berufsprofilkategorie auf Basisebene gehört, aus der folgenden Liste ausgewählt sind: Herzfrequenz, Atemfrequenz, GCS-Skala, AVPU-Skala, Kapillarnachfüllung, Zyanose, Blässe, Diaphorese/Fleckenbildung.

4. System nach einem der vorhergehenden Ansprüche, wobei die biometrischen Parameter, die von einem Bediener erfassbar sind, der zu einer Berufsprofilkategorie auf einer Ebene oberhalb der Basisebene gehört, aus der folgenden Liste ausgewählt sind: Blutdruck, Sauerstoffsättigung (SaO2), CO2-Wert am Ende der Ausatmung (ETCO2), Blutglukose, Körpertemperatur, Troponin, Blutsauerstoffpartialdruck, Blut-CO2-Partialdruck, Atemnot, sensorische Störungen, Sauerstofftherapiereaktion, Schockklassen von 1 bis 4, typischer Thoraxschmerz, kaltes Schwitzen, externe Blutung.

5. System nach einem der vorhergehenden Ansprüche, wobei der Prozessor der mobilen Vorrichtung programmiert ist, um einen sofortigen Transport eines Patienten zu Gesundheitseinrichtungen als Funktion zumindest des abgeleiteten Schweregrads des Zustands des Patienten zu arrangieren und anzufordern.

6. System nach einem der vorhergehenden Ansprüche, wobei das mindestens eine tragbare elektronische Gerät (5) programmiert ist, um die Werte der erfassten biometrischen Parameter periodisch zu aktualisieren und folglich den Schweregrad durch erneutes Ausführen des Programmcodes zu aktualisieren.

7. Verfahren zur Unterstützung eines Bedieners in einer Notfallsituation mit einem Patienten, umfassend die folgenden Schritte:
- Empfangen eines Identifikationscodes, der einem Bediener eindeutig zugewiesen ist;
- Erfassen eines oder mehrerer biometrischer Parameter eines Patienten, der von dem Bediener unterstützt wird, durch ein tragbares elektronisches Gerät (5);
- Empfangen des einen oder der mehreren biometrischen Parameter in einer mobilen Vorrichtung (4), die drahtlos mit dem mindestens einen tragbaren elektronischen Gerät (5) verbunden ist und einen Speicher einschließt, der mindestens einen Diagnosealgorithmus enthält, um einen Schweregrad des Zustands des Patienten abzuleiten;
- Vergleichen des einen oder der mehreren erfassten biometrischen Parameter mit jeweiligen vorbestimmten Wertebereichen;
- in einem Prozessor der mobilen Vorrichtung (4) Durchführen des mindestens einen Diagnosealgorithmus als Funktion der empfangenen biometrischen Parameter,
und um den Schweregrad mit einer entsprechenden Farbe zu assoziieren, die auf der Anzeige (8) der mobilen Vorrichtung (4) erscheint;
**dadurch gekennzeichnet, dass**
- das tragbare elektronische Gerät (5) mit einem Speicher und einem Prozessor versehen ist und durch einen Triage-Algorithmus, der von dem Prozessor ausgeführt wird, den Schweregrad des Patienten als Funktion einer Vielzahl von Parametern ableitet, von denen mindestens einige die biometrischen Parameter sind, die von dem mindestens einen tragbaren elektronischen Gerät (5) erfasst werden; und
dass das Verfahren den folgenden Schritt umfasst, der von einem Prozessor der mobilen Vorrichtung (4) durchgeführt wird:
- Zugreifen auf eine Datenbank, die eine Vielzahl von Identifikationscodes, die jeweils eindeutig einem Bediener (2) zugewiesen sind, und eine Vielzahl von Berufsprofilkategorien für den Bediener enthält;
- Auswählen der Berufsprofilkategorie, die dem erhaltenen Identifikationscode entspricht,
- Auswählen mindestens eines therapeutischen Vorgehens aus einer Vielzahl von vorbestimmten therapeutischen Vorgehen als Funktion der ausgewählten Berufsprofilkategorie und als Funktion des Schweregrads des Zustands des Patienten, der durch die mobile Vorrichtung abgeleitet wird, um dem Bediener praktische Hinweise über die zu befolgende Vorgehensweise zu geben, wobei das mindestens eine tragbare elektronische Gerät ferner eine Anzeige oder ein aus einer Entfernung sichtbares Element umfasst, das unter der Steuerung eines Prozessors des tragbaren elektronischen Geräts mit einer austauschbaren Farbe gefärbt werden kann, um einen Referenzcode hervorzuheben, der die Schwere der Zustände des Patienten darstellt, wie sie durch das tragbare elektronische Gerät abgeleitet werden.

8. Verfahren nach Anspruch 7, umfassend einen Schritt zum Arrangieren eines sofortigen Transports von Patienten zu Gesundheitseinrichtungen als Funktion mindestens des abgeleiteten Schweregrads des Zustands eines jeden Patienten.

9. Verfahren nach einem der Ansprüche 7 oder 8, wobei das mindestens eine tragbare elektronische Gerät (5) die Werte der erfassten biometrischen Parameter periodisch aktualisiert und folglich den Schweregrad durch erneutes Ausführen des Programmcodes aktualisiert.

10. Computerprogramm, umfassend computerausführbare Anweisungen zum Durchführen der Schritte durch den Prozessor der mobilen Vorrichtung nach einem der Ansprüche 7 bis 9, wenn sie auf der mobilen Vorrichtung des Systems nach einem der Ansprüche 1 bis 6 ausgeführt werden, und umfassend computerausführbare Anweisungen zum Durchführen der Schritte durch den Prozessor des mindestens einen elektronischen Geräts nach einem der Ansprüche 7 bis 9, wenn sie auf dem mindestens einen elektronischen Gerät des Systems nach einem der Ansprüche 1 bis 6 ausgeführt werden.

11. Datenspeichervorrichtung, die von einem Computer lesbar ist und das Computerprogramm nach Anspruch 10 enthält.

12. Datenfluss, der auf einen Computer heruntergeladen werden kann und das Computerprogramm nach Anspruch 10 darstellt.

## Revendications

1. Système (1) pour aider un opérateur (2) dans une situation d'urgence impliquant un patient (3), comprenant :
- au moins un appareil électronique portable (5) pouvant être positionné en correspondance du patient et comprenant un ou plusieurs capteurs (6) configurés pour détecter un ou plusieurs paramètres biométriques du patient (3) ;
- au moins un dispositif mobile (4) pouvant être connecté sans fil à l'au moins un appareil électronique portable (5) et configuré pour recevoir les paramètres biométriques détectés par l'au moins un appareil électronique portable, l'au moins un dispositif mobile (4) comprenant un accès à une mémoire pour stocker les paramètres biométriques détectés et comprenant un écran (8) et un processeur programmé pour recevoir et traiter les paramètres biométriques reçus, dans lequel la mémoire contient en outre au moins un algorithme de diagnostic pour déduire un niveau de gravité de l'état du patient, et dans lequel le processeur est configuré pour exécuter l'au moins un algorithme de diagnostic en fonction des paramètres biométriques reçus, et pour associer le niveau de gravité à une couleur correspondante apparaissant sur l'écran (8) du dispositif mobile (4) ;
- une banque de données accessible par l'au moins un dispositif mobile (4) et contenant une pluralité de codes d'identification, chacun attribué de manière unique à un opérateur (2),
dans lequel :
- l'au moins un appareil électronique portable (5) comprend une mémoire et un processeur, dans lequel la mémoire contient un code de programme représentant un algorithme de triage, exécuté par le processeur dudit au moins un appareil électronique portable et configuré pour déduire le niveau de gravité du patient en fonction d'une pluralité de paramètres, dont certains au moins sont les paramètres biométriques détectés par l'au moins un appareil électronique portable (5) ;
- la banque de données contient une pluralité de catégories de profils professionnels pour l'opérateur, dans lequel chaque code d'identification de chaque opérateur (2) est associé à l'une des catégories de profils professionnels ;
- l'au moins un dispositif mobile (4) comprend une interface d'échange de données configurée pour recevoir le code d'identification de l'opérateur, dans lequel la mémoire contient une pluralité de procédures thérapeutiques prédéterminées, et dans lequel le processeur du dispositif mobile (4) est programmé pour accéder à la banque de données et sélectionner la catégorie de profils professionnels correspondant au code d'identification reçu, et pour sélectionner au moins une procédure thérapeutique à partir de la pluralité en fonction de la catégorie de profils professionnels sélectionnée et en fonction du niveau de gravité de l'état du patient déduit par le dispositif mobile, de manière à fournir à l'opérateur des indications pratiques quant à la marche à suivre, dans lequel l'au moins un appareil électronique portable comprend en outre un écran ou un élément visible à distance qui peut être coloré avec une couleur interchangeable sous le contrôle d'un processeur de l'appareil électronique portable afin de mettre en évidence un code de référence représentant la gravité de l'état du patient telle que déduite par l'appareil électronique portable.

2. Système selon la revendication 1, comprenant un serveur contenant la banque de données et en communication avec le dispositif mobile par l'intermédiaire d'une connexion locale ou à distance.

3. Système selon l'une quelconque des revendications précédentes, dans lequel les paramètres biométriques détectables par un opérateur appartenant à une catégorie de profils professionnels au niveau de base sont choisis dans la liste suivante : fréquence cardiaque, fréquence respiratoire, échelle de Glasgow, échelle AVPU, temps de remplissage capillaire, cyanose, pâleur, diaphorèse/taches sur la peau.

4. Système selon l'une quelconque des revendications précédentes, dans lequel les paramètres biométriques détectables par un opérateur appartenant à une catégorie de profils professionnels à un niveau supérieur au niveau de base sont choisis dans la liste suivante : pression artérielle, saturation en oxygène (SaO2), valeur de CO2 en fin d'expiration (ETCO2), glycémie, température corporelle, troponine, pression partielle en oxygène du sang, pression partielle en CO2 du sang, détresse respiratoire, troubles sensoriels, réponse à l'oxygénothérapie, classes de chocs de 1 à 4, douleur thoracique typique, sueurs froides, hémorragies externes.

5. Système selon l'une quelconque des revendications précédentes, dans lequel le processeur du dispositif mobile est programmé pour organiser et demander le transport immédiat d'un patient vers des établissements de santé en fonction au moins du niveau de gravité déduit de l'état du patient.

6. Système selon l'une quelconque des revendications précédentes, dans lequel l'au moins un appareil électronique portable (5) est programmé pour mettre à jour périodiquement les valeurs des paramètres biométriques détectés et, par conséquent, pour mettre à jour le niveau de gravité en exécutant à nouveau le code de programme.

7. Procédé pour aider un opérateur dans une situation d'urgence impliquant un patient, comprenant les étapes suivantes :
- recevoir un code d'identification attribué de manière unique à un opérateur ;
- détecter un ou plusieurs paramètres biométriques d'un patient assisté par l'opérateur, par l'intermédiaire d'un appareil électronique portable (5) ;
- recevoir l'un ou plusieurs paramètres biométriques dans un dispositif mobile (4), connecté sans fil à l'au moins un appareil électronique portable (5) et incluant une mémoire contenant au moins un algorithme de diagnostic pour déduire un niveau de gravité de l'état du patient ;
- comparer l'un ou plusieurs paramètres biométriques détectés à des plages de valeurs prédéterminées respectives ;
- dans un processeur du dispositif mobile (4), exécuter l'au moins un algorithme de diagnostic en fonction des paramètres biométriques reçus,
et associer le niveau de gravité à une couleur correspondante apparaissant sur l'écran (8) du dispositif mobile (4) ;
**caractérisé en ce que**
- l'appareil électronique portable (5) est doté d'une mémoire et d'un processeur et déduit, par l'intermédiaire d'un algorithme de triage exécuté par le processeur, le niveau de gravité du patient en fonction d'une pluralité de paramètres, dont certains au moins sont des paramètres biométriques détectés par l'au moins un appareil électronique portable (5) ; et
**en ce que** le procédé comprend les étapes suivantes, exécutées par un processeur du dispositif mobile (4) :
- accéder à une banque de données contenant une pluralité de codes d'identification, chacun attribué de manière unique à un opérateur (2), et une pluralité de catégories de profils professionnels pour l'opérateur ;
- sélectionner la catégorie de profils professionnels correspondant au code d'identification reçu,
- sélectionner au moins une procédure thérapeutique parmi une pluralité de procédures thérapeutiques prédéterminées, en fonction de la catégorie de profils professionnels sélectionnée et en fonction du niveau de gravité de l'état du patient déduit par le dispositif mobile, de manière à fournir à l'opérateur des indications pratiques sur la marche à suivre,
dans lequel l'au moins un appareil électronique portable comprend en outre un écran ou un élément visible à distance qui peut être coloré avec une couleur interchangeable sous le contrôle d'un processeur de l'appareil électronique portable afin de mettre en évidence un code de référence représentant la gravité de l'état du patient telle que déduite par l'appareil électronique portable.

8. Procédé selon la revendication 7, comprenant une étape consistant à organiser le transport immédiat des patients vers des établissements de santé en fonction au moins du niveau de gravité déduit de l'état de chaque patient.

9. Procédé selon l'une quelconque des revendications 7 ou 8, dans lequel l'au moins un appareil électronique portable (5) met périodiquement à jour les valeurs des paramètres biométriques détectés et, par conséquent, met à jour le niveau de gravité en exécutant à nouveau le code de programme.

10. Programme informatique comprenant des instructions exécutables par ordinateur pour l'exécution des étapes par le processeur du dispositif mobile, selon l'une quelconque des revendications 7 à 9, lorsqu'elles sont exécutées sur le dispositif mobile du système selon l'une quelconque des revendications 1 à 6, et comprenant des instructions exécutables par ordinateur pour l'exécution des étapes par le processeur dudit au moins un appareil électronique, selon l'une quelconque des revendications 7 à 9, lorsqu'elles sont exécutées sur ledit au moins un appareil électronique du système selon l'une quelconque des revendications 1 à 6.

11. Dispositif de stockage de données lisible par un ordinateur et contenant le programme informatique selon la revendication 10.

12. Flux de données téléchargeable sur un ordinateur et représentant le programme informatique selon la revendication 10.
